# EUROPEAN PATENT SPECIFICATION

(11) **EP 0 933 138 B1**
(45) Date of publication and mention of the grant of the patent: **03.03.2004**
(21) Application number: 99105093.1
(22) Date of filing: 09.04.1993
(51) Int. Cl.: B05B 17/06

(54) **Ultrasonic atomizer**
Ultraschallzerstäuber
Atomiseur à ultrasons

(30) Priority: 09.04.1992 JP 8846392; 10.04.1992 JP 9085292; 14.04.1992 JP 9404392
(43) Date of publication of application: 04.08.1999
(62) Divisional of application: 93908070.1
(73) Proprietor: Omron Healthcare Co., Ltd., Kyoto 600-0084 (JP)
(72) Inventor: Yamammoto, Hirohito, Yamanouchi, Ukyo-ku, Kyoto-shi, Kyoto615 (JP); Asai, Kei, Yamanouchi, Ukyo-ku, Kyoto-shi, Kyoto615 (JP); Obata, Masaru, Yamanouchi, Ukyo-ku, Kyoto-shi, Kyoto615 (JP)
(74) Representative: WILHELMS, KILIAN & PARTNER Patentanwälte

(56) References cited:
- US-A- 4 301 093
- US-A- 4 793 339
- US-A- 4 850 534

## Description

### Technical Field

This invention relates to an ultrasonic atomizer.

### Background Art

An ultrasonic atomizer according to the preamble of claim 1 is known from US-A- 4 850 534.

### Disclosure of the Invention

An object of the present invention is to provide an ultrasonic atomizer enabling effective utilisation of liquid with which it has been filled.

The present invention provides an ultrasonic atomizer in which liquid inside the liquid vessel can be utilized without any being left unused.

The present invention is as defined in claim 1, i.e., in an ultrasonic atomizer comprising a liquid vessel accommodating a liquid to be atomized, and an ultrasonic pump having a pump shaft formed to have a pump bore passing through it axially and having open upper and lower ends, and an ultrasonic vibrator mounted on the pump shaft, a lower end of the pump shaft is disposed in close proximity to a bottom surface or side surface of the liquid vessel in such a manner that residual liquid remaining inside the liquid vessel is pumped upon attaching itself to the lower end of the pump shaft by surface tension.

In a preferred embodiment, the liquid vessel is formed to have a recess for collecting the residual liquid remaining inside the liquid vessel, and the lower end of the pump shaft is disposed so as to face the recess.

Even if the amount of liquid remaining in the liquid vessel is small, the liquid attaches itself to the lower end of the pump shaft and is pumped by virtue of surface tension and ultrasonic vibration so that almost all of the liquid is used for spraying purposes. This is particularly effective when a costly medicine is used as the liquid.

### Brief Description of the Drawings

Fig. 1 is a side view, partially cut away, showing the overall construction of an ultrasonic atomizer;
Fig. 2 is an enlarged sectional view showing the principal mechanical construction of the ultrasonic atomizer;
Fig. 3 is an enlarged plan view of a mesh plate;
Fig. 4 is an enlarged plan view showing another example of a mesh plate;
Fig. 5 is an enlarged plan view showing yet another example of a mesh plate;
Fig. 6 is an enlarged sectional view showing a portion of a mesh plate;
Fig. 7 is an enlarged sectional view showing a portion of another example of a mesh plate;
Fig. 8 is an enlarged sectional view showing a portion of a cap, a mesh plate and a horn;
Fig. 9 is an enlarged sectional view showing another example of a portion of a cap, a mesh plate and a horn;
Fig. 10 is an enlarged sectional view showing yet another example of a portion of a cap, a mesh plate and a horn;
Fig. 11 is a perspective view showing another example of a mesh plate;
Figs. 12a through 12c show another example of a mesh plate, in which Fig. 12a is a plan view, Fig. 12b a sectional view taken along line b-b in Fig. 12a and Fig. 12c a side view;
Fig. 13 is an enlarged sectional view showing another example of a compression spring;
Fig. 14 is an enlarged sectional view showing another example of a biasing resilient member;
Fig. 15 is an enlarged sectional view showing an example in which an annular plate is attached to a mesh plate;
Fig. 16 is an enlarged sectional view showing another example in which an annular plate is attached to a mesh plate;
Fig. 17 is an enlarged sectional view showing an example in which a spacer is combined with a mesh plate;
Fig. 18 is an enlarged plan view of the spacer;
Fig. 19 is an enlarged plan view showing another example of a spacer;
Fig. 20 is a partially cut-away plan view of a liquid vessel; and
Fig. 21 is a sectional view taken along line XXI-XXI of Fig. 20.

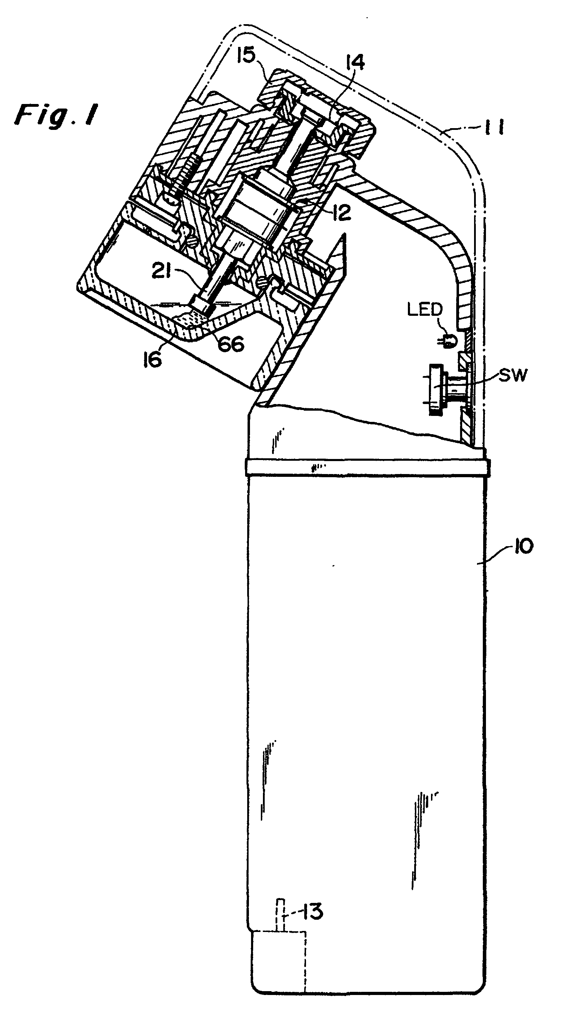

Fig. 1 illustrates the overall construction of an ultrasonic atomizer. In order to facilitate an understanding of the invention, the ultrasonic atomizer is shown somewhat larger than actual size.

The lower half portion of the ultrasonic atomizer extends vertically in the form generally of a rectangle and has a cross section which is nearly a regular square, and the upper half portion is curved upward at an incline so as to point in the forward direction. The upper half portion, especially the uppermost part of the device, is provided with the principal mechanical components such as an ultrasonic pump 12, a mesh plate 14 for atomizing the liquid pumped up by the pump, a cap 15 holding the mesh plate 14, and a liquid vessel 16 accommodating a liquid (medicine or the like). These will be described later. The rear side of the upper half portion of the housing is covered by a freely detachable cover indicated by the chain lines. The cover is detached when the ultrasonic atomizer is used.

Electric circuitry composed of a drive circuit of the ultrasonic pump inclusive of an ultrasonic vibrator circuit, a control circuit having various operating modes, and a power supply circuit is incorporated within the lower half portion of the housing 10. A switch SW operated by the user and a display device (light-emitting diode) LED are provided on the back side of the upper half portion covered by the cover 11. The ultrasonic atomizer has an internal chargeable battery. A jack 13 into which a plug led out from an external AC adapter or the like is plugged in order to charge the battery is provided in a cavity in the lower portion of the housing 10.

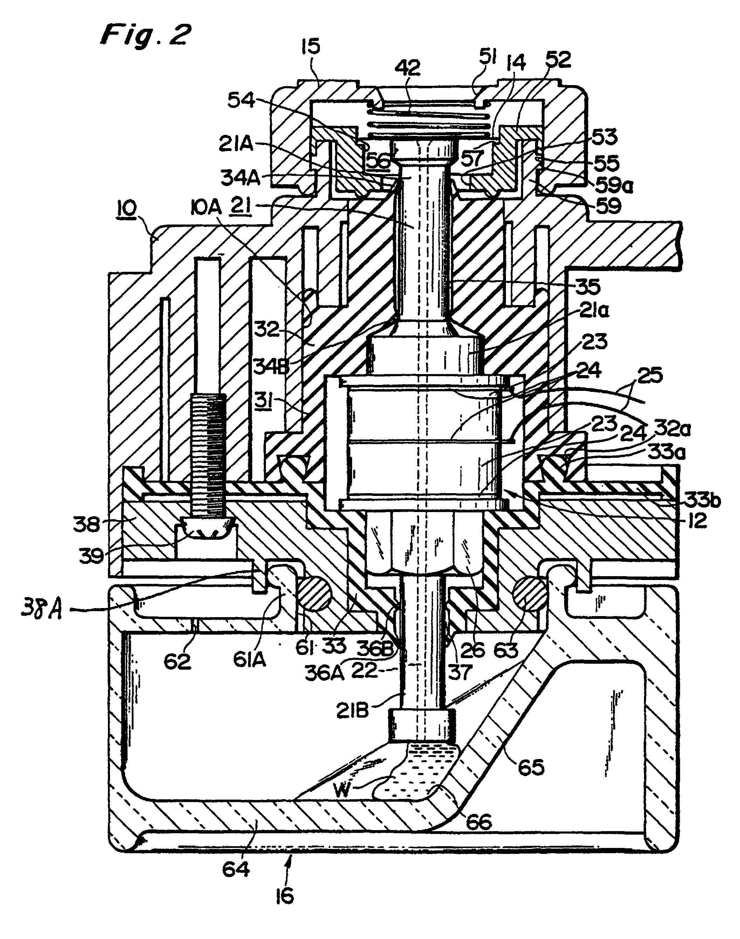

Fig. 2 illustrates, in larger size, the principal mechanical components provided in the upper half portion of the housing 10.

The ultrasonic pump 12 comprises a metallic pump shaft 21 having a small (diameter of not more than 1 mm) pump bore 22 passing through it in the axial direction, and two piezoelectric elements (ultrasonic vibrators) 23 secured to the pump shaft 21 substantially at its midpoint along the length direction thereof. A flange 21a is formed as an integral part of the pump shaft 21 at a portion of the pump shaft 21 somewhat above its midpoint. The piezoelectric elements 23 are annular in shape and are fitted over the pump shaft 21. Annular electrode plates 24 are provided on both sides of the piezoelectric elements 23. A nut 26 is screwed onto male threads formed on the pump shaft 21 so that the piezoelectric elements 23 and electrode plates 24 are firmly secured to the pump shaft 21 between the flange 21a and nut 26. A high-frequency (ultra-high-frequency) voltage from the above-described drive circuit is applied to the electrodes 24 through lead wires 25 so that the piezoelectric elements 23 may vibrate ultrasonically mainly in the axial direction of the pump shaft 21.

A pump bore 22 opens in the lower end face of the pump shaft 21 as well as in the upper end face of the pump shaft 21. A lower end portion 21B of the pump shaft 21 projects into the liquid vessel 16 and is submerged within the liquid inside the vessel 16. The pump shaft 21 has an upper end portion 21A serving as a horn, with the diameter of the upper end being made somewhat larger. The ultrasonic vibration of the piezoelectric elements 23 is transmitted to the pump shaft 21 so that the pump shaft 21 also undergoes ultrasonic vibration in the axial direction, as a result of which the liquid inside the liquid vessel 16 rises within the pump bore 22 of the pump shaft 21. It is believed that this is ascribable to the force applied by the ultrasonic vibration, the surface tension of the liquid and the negative pressure generated inside the pump bore 22.

The above-described ultrasonic pump 12 is encircled by a waterproof bush 31 comprising a resilient body (rubber, for example) and is secured to the upper half portion of the housing 10 via the bush 31.

The bush 31 is constituted by an upper half body 32 and a lower half body 33. A space which accommodates the ultrasonic pump 12 is formed inside the upper half body 32 and lower half body 33. An annular groove 32a is formed in the bottom side of the upper half body 32 and an annular projection 33a is formed in the top side of the lower half body 33. The two bodies 32, 33 are joined to construct the bush 31 by mating the projection 33a with the groove 32a. The upper portion of the lower half body 33 is further formed to have an outwardly extending flange 33b. The upper half body 32 is tightly fitted into an accommodating recess 10A formed in the housing 10. The lower half body 33 is supported inside a recess of a retaining member 38. The latter is secured to the housing 10 at three locations (only one of which is shown) by means of screws 39. Thus, the entire portion of the pump shaft 21, with the exception of an upper end portion 21A and lower end portion 21B thereof, of the ultrasonic pump 12 is covered water tightly by the bush 31, and the pump shaft is secured to the housing 10.

Upper and lower portions of an inner circumferential surface of a cylindrical portion formed on the upper half body 32 of the bush and penetrated by the pump shaft 21 are formed to have inwardly projecting annular seal lips 34A, 34Bas integral parts of the upper half body 32 of the bush. The annular seal lips 34A, 34B are in intimate contact with the outer circumferential surface of the pump shaft 21 and maintain liquid tightness. Between the seal lip 34A of the upper portion and the seal lip 34B of the lower portion, a small gap 35 is formed between the inner circumferential surface of the cylindrical portion of the upper half body 32 of the bush and the outer circumferential surface of the pump shaft 21. Since the area of contact between the upper half body 32 of the bush and the upper portion (horn) 21A of the pump shaft 21 is small, ultrasonic vibration of a large amplitude can be obtained at the upper portion of the pump shaft 21. The middle (flange 21a, vibrators 23, nut 26, etc.) of the ultrasonic pump 12 where the amplitude of vibration is small is held tightly by the bush 31.

Similarly, with regard to the lower half body 33 of the bush, upper and lower portions of the inner circumferential surface of a cylindrical portion of the lower half body penetrated by the pump shaft 21 are formed to have inwardly projecting annular seal lips 36B, 36A as integral parts of the lower half body 33. The seal lips 36A, 36B are in intimate contact with the outer circumferential surface of the lower portion of pump shaft 21, whereby liquid tightness is maintained. Further, between the upper and lower seal lips 36B, 36A, a small gap 37 is formed between the lower half body 33 and the pump shaft 21 to assure large ultrasonic vibration.

The periphery of the housing at a position where the upper end portion 21A of the pump shaft 21 protrudes is integrally formed to have a cylindrical portion (annular projection) 59, which is low in height, for the purpose of attaching the cap 15. The outer circumferential portion of the cylindrical portion 59 is formed to have projections 59a at two locations.

The cap 15 is formed to have a spray port 51 in its top side. The bottom side of the cap is open. A base 52 is fitted into the cap 15. The base 52 is formed to have a hole 53 through which the upper end portion of the pump shaft 21 is loosely passed, and the outer side of the base is formed to have an annular groove which receives the cylindrical portion 59. The inner circumferential surface of the cap 15, which is formed by the base 52 and cap 15, is formed to have vertical grooves (not shown in Fig. 2) through which the projections 59a are passed, as well as a groove 55 leading to the upper ends of the vertical grooves and inclined diagonally upward at a slight angle. Accordingly, the cap 15 is secured to the cylindrical portion 59 (this is the state shown in Fig. 2) by placing the cap on the cylindrical portion 59 at a position where the projections 59a coincide with the vertical grooves and then turning the cap 15 slightly, whereby the projections 59a are fitted into the groove 55 and moved into the groove 55. Thus, the cap 15 is capable of being attached to the housing 10 in a freely detachable manner. It goes without saying that the arrangement for attaching the cap 15 to the cylindrical portion 59a is not limited to that described above. The cap can be attached by screws or simply by mating projections with recesses.

The upper portion of the base 52 of cap 15 is further formed to include an annular step 57 at a position facing a recess 54. The mesh plate 14, which is provided with a multiplicity of minute holes, is placed upon the annular step 57. A compression spring 42 is provided between the periphery of the mesh plate 14 and the periphery of the spray port 51 of the cap 15. In a state in which the cap 15 has been detached from the housing 10, the mesh plate 14 is pressed against step 57 at the periphery of the mesh plate by the compression spring 42. When the cap 15 is attached to the cylindrical portion 59 of the housing 10, the upper end face of the pump shaft 21 abuts against the central portion of the mesh plate 14 and the periphery of the mesh plate 14 lifts up slightly from the step 57. The mesh plate 14 is biased in the direction of the upper end face of the pump shaft 21 at all times by the spring 42 so that the mesh plate 14 will vibrate by following up the vertical vibration of the horn 21A. Liquid which has risen through the pump bore 22 of the pump shaft 21 is atomized very finely through the mesh plate 14 and is sprayed to the outside from the spray port 51. The details of the mesh plate 14 as well as the relationship among the mesh plate 14, the spring 42 and the upper end face of the pump shaft 21 will be described in detail later.

The mesh plate 14 is attached to the cap 15. Since the cap 15 is freely attachable and detachable with respect to the housing 10, it is easy to clean or replace the mesh plate 15 (where replacement can mean replacement of the mesh and cap). Further, positional adjustment of the mesh plate 14 relative to the upper end face of the pump shaft 21 is made unnecessary.

A space delimited by the top side of the upper half body 32 of the bush inclusive of the annular seal lip 34A, the outer circumferential surface of the horn 21A, the hole 53 in the base 52 and the recess 54 serves as a liquid reservoir 56. In a case where the amount of liquid pumped to the upper end face of the horn 21A by the ultrasonic pump 12 is greater than the amount of liquid atomized, as a result of which the liquid overflows from the upper end face of the horn 21A, and in a case where the user accidentally allows to drip liquid, any liquid will collect temporarily in the liquid reservoir 56. Owing to the ultrasonic vibration of the horn 21A, however, the liquid is pumped up to the bottom side of the mesh plate 14 and atomized. A decline in atomization performance or instability thereof ascribed to attenuation of the vibrational amplitude of the horn 21A or to some other cause can thus be prevented. Further, since liquid which has overflowed from the upper end face of the horn 21A is prevented from flowing out to the exterior of the housing 10, there is no risk of the user's fingers being soiled and loss of costly medicine can be prevented.

The retaining member 38 projects downwardly in the form of a cylinder from the portion surrounding the lower half body 33 of the bush and the periphery of this cylindrical projection is provided with an O-ring 63. Further, an annular projection 38A is formed on the bottom side of the retaining member 38 on the outer side of the cylindrical projection. The annular projection 38A preferably is partially broken off at a plurality of locations. The liquid vessel 16, meanwhile, is formed from a transparent material. The top side of the vessel has an opening 61 the periphery of which projects slightly in the form of a cylinder (the periphery of the cylindrical opening is indicated at numeral 61A). By inserting the cylindrical periphery 61A of the liquid vessel 16 between the O-ring 63 and the annular projection 38A, the vessel 16 is fixedly attached to the bottom side of the retaining member 38. The liquid vessel may thus be freely attached to and detached from the housing 10. Since the vessel 16 is transparent, the amount of liquid inside can be confirmed visually from the outside. The top side of the vessel 16 is provided with a small hole 62 communicating with the atmosphere. This is to prevent negative pressure from being produced inside the liquid vessel as a result of the liquid in the liquid vessel 16 being pumped by the ultrasonic pump 12. The details of the structure of the vessel 16 and the advantages thereof will be described later.

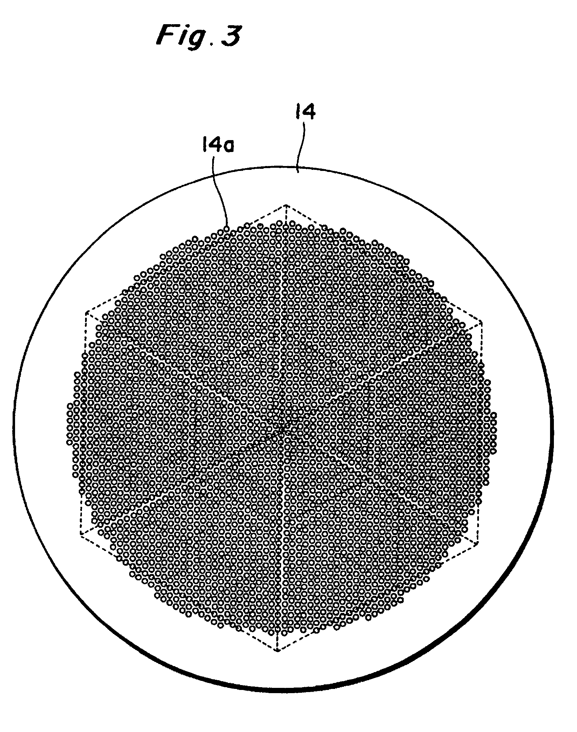

Fig. 3 is an enlarged plan view of the mesh plate 14. The mesh plate 14 is provided with a multiplicity of minute holes 14a, as mentioned above.

The multiplicity of holes 14a preferably are formed at equal intervals along the sides of a number of regular hexagons drawn with their centers on the center of the circular mesh plate 14, as well as at the apices of the hexagons. The lengths of the diagonals of the number of regular hexagons vary at fixed lengths from one hexagon to the next. The number of minute holes 14a along one side of a regular hexagon differs from the number along one side of the adjacent hexagon by one. If this arrangement is adopted, the number of minute holes 14a per unit area is maximized and the amount of liquid atomized is increased as a result.

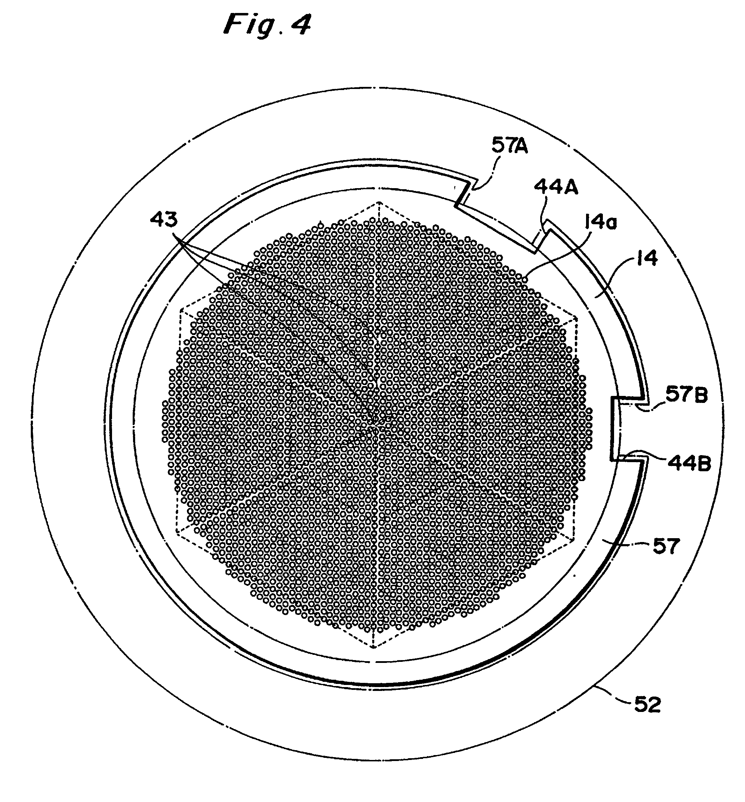

Fig. 4 illustrates another example of the mesh plate 14. This mesh plate has two characterized features in addition to those mentioned above.

One feature is that blank portions 43 devoid of the minute holes 14a are provided. There are a total of three blank portions 43 in Fig. 4, namely at a point at the center and at two points established at intervals of five minute holes from the center along one portion of a diagonal of the regular hexagons.

The mesh plate is formed to have a multiplicity of minute holes. In the process for manufacturing the mesh plate, at the time of delivery and at other necessary times, inspection is required in order to determine whether the minute holes have been formed to the stipulated size and shape. Since it is virtually impossible to measure the diameters of all of the minute holes in this inspection, only specific minute holes are inspected. Since a change in the positions of minute holes to be inspected from one inspection to the next is undesirable, it is required that minute holes located at specific positions at all times be examined. If the blank portions 43 are provided as set forth above, minute holes that are to be examined can be determined using the blank portions 43 as a reference. For example, minute holes adjacent to and located on the outer side of the blank portions 43 may be examined. In this way the minute holes to undergo examination can be specified.

The other feature is that at least two types of cut-outs 44A, 44B of different size are formed at two respective locations (positions having point symmetry about the center of the mesh plate are excluded) on the periphery of the mesh plate 14. As will be described later, the mesh plate 14 has a front surface and a back surface (or a top surface and a bottom surface), and the mesh plate 14 must be installed in the cap 15 in such a manner that one surface of the mesh plate comes into surface contact with the upper end face of the horn 21A. Projections 57A, 57B of different size are provided on the step portion 57 of the base 52 of cap 15 and mate perfectly with the cut-outs 44A, 44B, respectively, of the mesh plate 14 when the mesh plate 14 has been placed in the correct surface orientation. If the cut-outs 44A, 44B are situated so as to mate with the projections 57A, 57B, the mesh plate 14 will be installed in the cap 15 with its surfaces pointing in the correct directions.

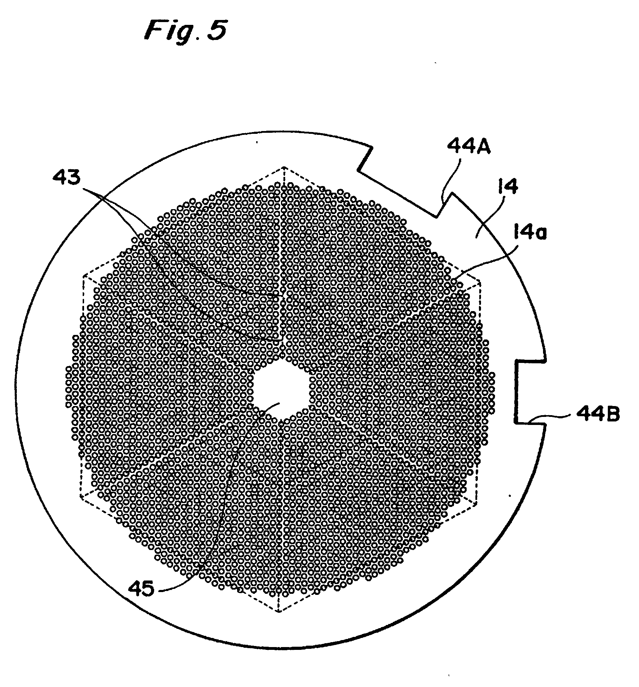

A mesh plate 14 illustrated in Fig. 5 has still another characterizing feature. This is the fact that a comparatively large closed portion 45 is provided at the center of the mesh plate 14. No minute holes are formed in the closed portion 45. When the cap 15 having the mesh plate 14 installed therein has been fitted on the housing 10, the closed portion 45 opposes the opening in the pump bore 22 open at the upper end face of the horn 21A and acts to periodically close the opening as the opening vibrates. As a result, the valve action (described below) of the mesh plate 14 is reinforced so that more efficient pumping and spraying can be expected. The closed portion 45 should be large enough to close the opening in the upper end of the horn 21A. However, since the substantial area over which the minute holes may be provided is decreased if the closed portion is made too large, the closed portion should be sized so as not to have a deleterious effect upon the atomizing action. It goes without saying that in a case where the opening in the upper end of the horn 21A is not at the center but is offset to one side, the closed portion 45 also is provided in the mesh plate 14 at a position offset from the center thereof so that it will oppose the opening in the upper end of the horn.

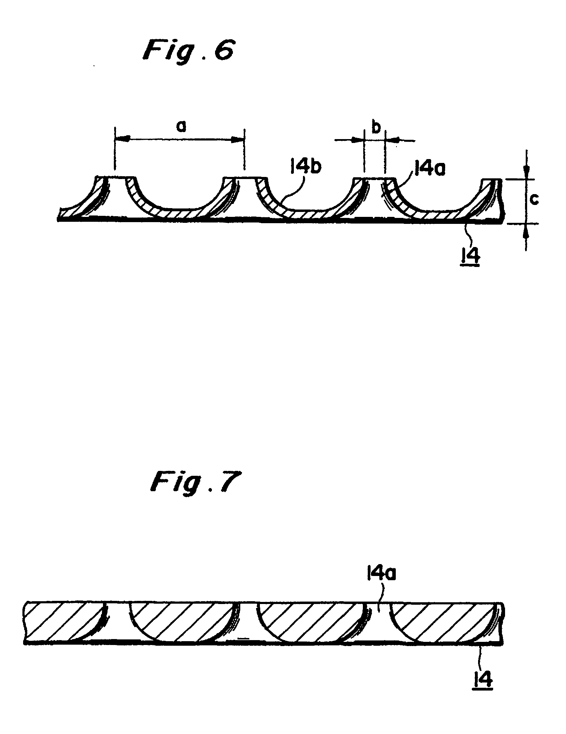

Fig. 6 illustrates a portion of the cross section of the mesh plate in enlarged form.

The diameter of the circular minute holes 14a is small at the top surface of the mesh plate 14. The holes become successively wider and the diameter successively larger toward the bottom surface of the mesh plate. A recess or groove 14b opening toward the top surface is formed on the periphery of each minute hole 14a, namely between the minute holes 14a. As a result, a shape is obtained in which the rim defining each minute hole 14a projects in the upward direction. The mesh plate 14 is installed in the cap 15 in such a manner that the bottom surface of the mesh plate 14 opposes the upper end face of the horn 21A while the top surface opposes the spraying port 51 of the cap 15. This mesh plate 14 is characterized in that a comparatively large strength is obtained without making the plate thick.

As for an example of the dimensions of the various portions constituting the mesh plate having the sectional configuration shown in Fig. 6, the pitch a of the minute holes 14a is 50 ∼ 150 µm (a pitch on the order of 100 µm is suitable), the diameter b of the opening of each minute hole 14a in the top surface is on the order of 4.5 ± 1 µm, the thickness c of the mesh plate 14 is on the order of 30 ∼ 100 µm (a thickness on the order of 50 µm is suitable). Such a mesh plate can be fabricated by combining photoetching and electroforming techniques. Specifically, a number of electrodes are formed on an insulative plate by photoetching so as to correspond to the positions of the centers of the recesses 14b. This plate is adopted as a first negative. Next, a first metal is deposited on the electrodes by electroforming in such a manner that mutually independent mountains are formed. The resulting plate is adopted as a second negative. A second metal is further built up uniformly on each mountain of the second negative by electroforming. If the second negative is then peeled off the deposited second metal, a mesh plate comprising the layer of the second metal will be obtained. If it is acceptable for the minute holes to be comparatively large, the mesh plate having the shape shown in Fig. 6 can be fabricated by press work or injection molding.

Fig. 7 shows another example of the mesh plate, in which the cross section is illustrated in enlarged form. Each of the multiplicity of minute holes 14a flares downwardly in such a manner that the diameter of the circular minute holes 14a is small at the top surface of the mesh plate 13 and becomes successively larger toward the bottom surface. Such a mesh plate is known in the art. The material may be metal or a plastic film. The minute holes need not be circular.

The directions in which the surfaces point when the mesh plate has been mounted in the cap is important because the diameters of the minute holes at the top surface and bottom surface differ.

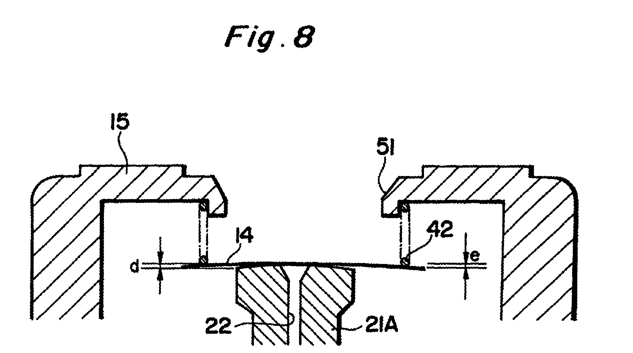

Fig. 8 illustrates, in enlarged form, the shape of the upper end face of the horn 21A and the relationship among the mesh plate 14, the compression spring 42 and the cap 15.

The upper end face of the horn 21A is formed so as to be slightly and smoothly curved (spherically) in such a manner that the end face protrudes upwardly to the maximum extent at the center where the pump bore 22 opens. The mesh plate 14 is held fast at its periphery by the compression spring 42, as a result of which the mesh plate is caused to flex slightly. By way of example, the difference d between the highest and lowest points of the upper end face of the horn 21A is on the order of 20 ∼ 50 µm, the amount of flexure of the mesh plate 14 is on the order of 5 ∼ 10 µm, and the degree of curvature of the upper end face of the horn 21A is greater than the degree of curvature of the mesh plate 14 caused by flexing. It is important that the vicinity of the opening to the pump bore 22 in the upper end face of the horn be pressed by the mesh plate under the force of the compression spring 42. The degree of curvature of the upper end face of the horn, the strength of the mesh plate and the spring force of the compression spring are selected so as to produce this relationship.

The vertical (up-and-down) vibration of the horn 12A is accompanied by up-and-down vibration of the mesh plate 14, as mentioned above. Though the minute holes 14a are formed in the mesh plate 14, overall the mesh plate 14 acts as a valve which, by vibrating, opens and closes the opening in the upper end of the pump bore 22. Owing to this valve action of the mesh plate 14, liquid pumped up from the pump bore spreads over the upper end face of the horn 21A to form a liquid film when the valve is opened. When the valve is closed, the pumping action is suppressed and the above-mentioned liquid film is atomized through the mesh plate 14. In other words, pumping and atomization are performed alternately so that the amount of liquid pumped and the amount of liquid atomized balance each other, whereby an efficient, stabilized spraying action is achieved. According to tests and experiments carried out by the inventors, a spraying operation exhibiting stable performance was achieved. At present the reasons are believed to be as set forth above. With a mesh plate having the closed portion 45, as shown in Fig. 5, the above-described valve operation can be expected to be attained with even greater efficiency. The pumping effect produced by the valve action of the mesh plate is particularly important at the beginning of the spraying operation.

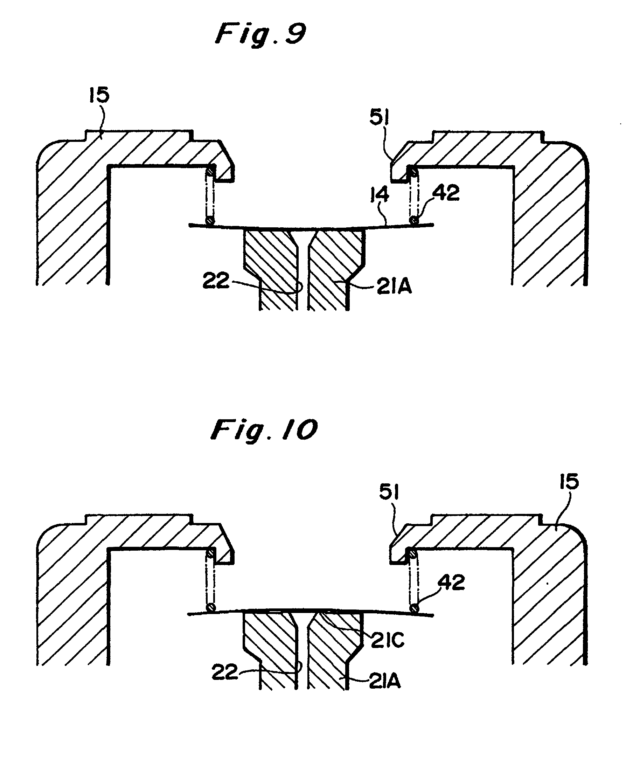

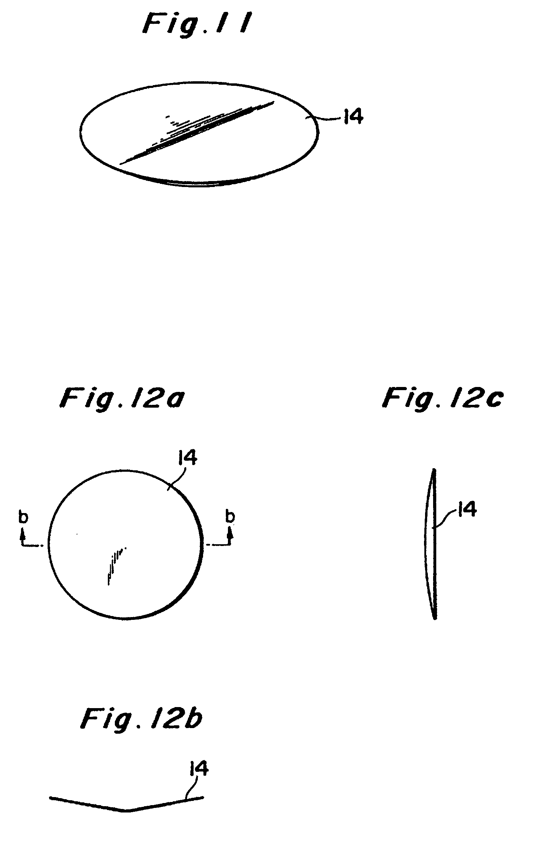

Fig. 9 illustrates another embodiment. Here the upper end face of the horn 21A is flat and the mesh plate 14 is curved so as to project downward. Fig. 11 is a perspective view of the mesh plate 14. Another example of a mesh plate usable in the arrangement shown in Fig. 9 is illustrated in Figs. 12a through 12c. Here the mesh plate 14 is folded slightly and smoothly along a boundary which is a straight line passing through the center of the mesh plate. The minute holes are deleted from the illustrations in Fig. 11 and in Figs. 12a through 12c.

Fig. 10 illustrates yet another embodiment. Here the portion of the upper end face of horn 21A surrounding the opening in the upper end of the pump bore 22 protrudes slightly (the protruding portion is indicated at 21C).

In all of the examples described above, the diameter of the coil compression spring is set to be the same at all portions. The arrangement is such that the compression spring urges the periphery of the mesh plate extending outwardly of the upper end face of the horn. As a consequence, the mesh plate is caused to curve into an upwardly directed projection, and hence there is the possibility that the mesh plate will not be able to close off the opening in the upper end of the pump bore. In order to arrange it so that the opening in the upper end of the pump bore can be constrained by the mesh plate, the upper end face of the horn is curved so as to project upward, a projection is formed or the mesh plate is curved into a downwardly directly projection.

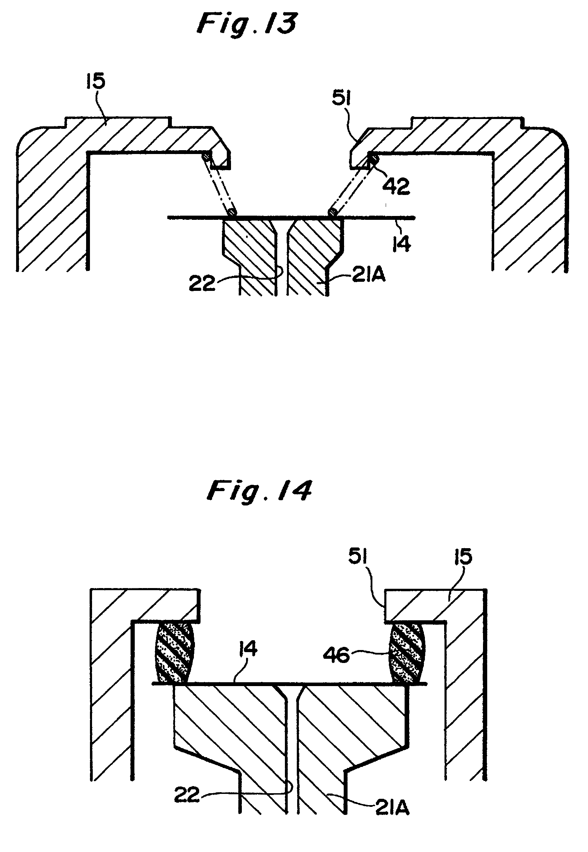

By contrast, when a compression spring according to a modification shown in Fig. 13 is used, it is unnecessary to work the upper end face of the horn or the mesh plate. Here the upper end face of the horn is flat and the mesh plate 14 is flat as well. The compression spring 42 has a small diameter at its lower portion, with the diameter of the spring growing larger from the bottom to the top. The compression spring 42 urges the mesh plate 14 at the portion thereof on the upper end face of the horn 21A.

Fig. 14 illustrates another example of the biasing resilient member. Here an annular sponge 46 is used instead of the spring.

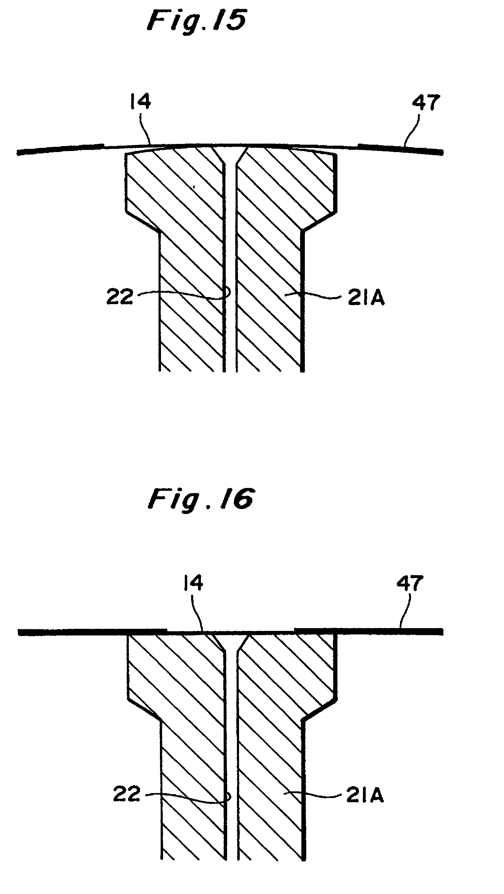

With the compression spring described above, the biasing force applied to the mesh plate will not necessarily be uniform. Accordingly, as shown in Fig. 15, it is recommended that an annular plate 47 simply be placed on the periphery of the mesh plate 14 or that the annular plate be fixed to periphery of the mesh plate 14 by bonding or welding, with the annular plate 47 being urged from above by the compression spring.

Fig. 16 shows an example in which the width of the annular plate 47 is enlarged so that the inner circumferential portion thereof engages the upper end face of the horn. In this case the upper end face of the horn 21A may be flat.

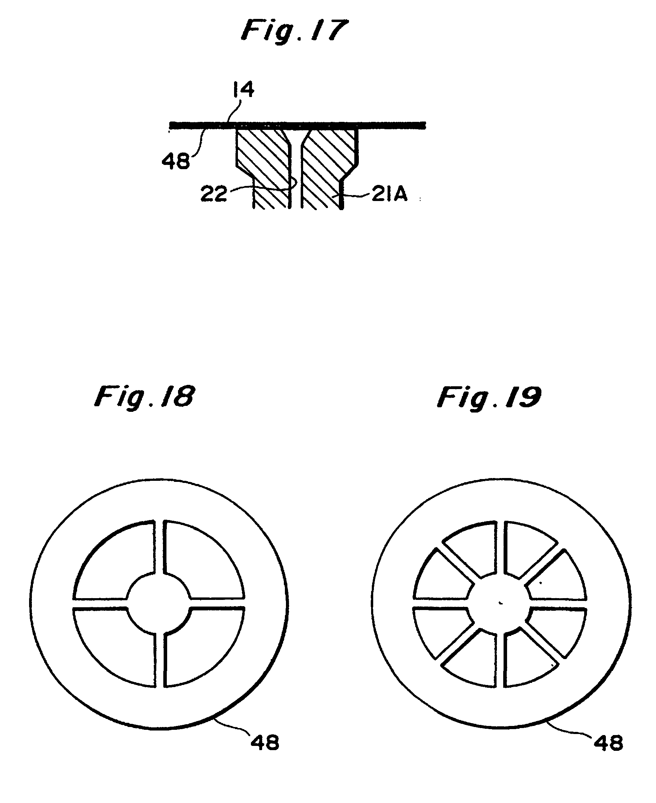

Fig. 17 illustrates an example in which a spacer 48 instead of the annular plate 47 is interposed between the mesh plate 14 and the upper end face of the horn. As shown in Fig. 18 or 19, the spacer 48 is integrally formed to include a small circular portion at its center, an annular portion along its circumference and radiating connecting spokes connecting these two portions. The small circular portion at the center should be sized so as to cover the opening of the pump bore 22 in the upper end face of the horn. The upper end face of the horn 21A may be flat. The annular plate 47 and spacer 48 may both be made of metal or plastic.

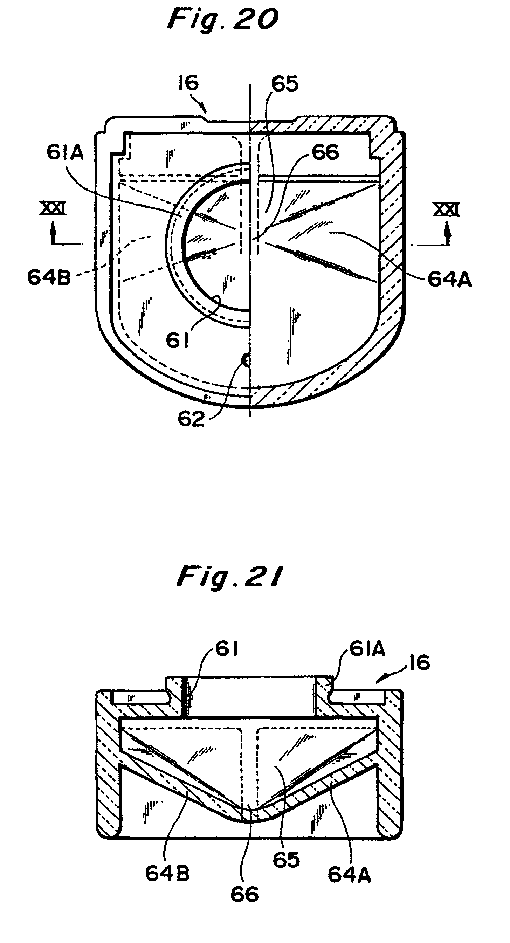

The shape of the liquid vessel 16 will now be described with reference to Figs. 2, 20 and 21.

The liquid vessel 16 has a rear wall 65 sloping downward toward the front. Further, both side portions 64A, 64B at the rear of a bottom wall 64 also slope upwardly toward the sides of the vessel. Since the principal mechanical portions of the ultrasonic atomizer are provided in an attitude in which they are inclined downwardly and forwardly, as shown in Fig. 1, the front portion of the bottom wall 64 is inclined downwardly and rearwardly in the state where the liquid vessel 16 is attached. In this way the liquid vessel 16 is formed to have its deepest recess 66, which is delimited by four inclined surfaces 64, 64A, 64B and 65. The lower end portion 21B of the pump shaft 21 is situated directly above the recess 66 in close proximity thereto and in fairly close proximity to the rear wall 65. By way of example, the distance between the lower end face of the pump shaft 21 and the bottom of the recess 66 is on the order of 2 ∼ 3 mm, and the distance between the rear wall 65 and the part of the peripheral surface of the lower end of the pump shaft 21 that is nearest to the rear wall 65 is on the order of 1 mm.

In use of the ultrasonic atomizer, the liquid vessel 16 is filled with an appropriate amount of liquid, i.e., to such an extent that the liquid will not overflow. The liquid in the liquid vessel 16 decreases as the device is operated to spray the liquid. When the amount of liquid becomes small, the liquid collects in the deepest recess 66. When this occurs, a small amount of the liquid attaches itself to the lower end of the pump shaft 21, as shown at W in Fig. 2, and the liquid is pumped and sprayed to the last drop, owing to the energy of ultrasonic vibration (negative pressure) and the surface tension of the liquid. Thus, all of the liquid introduced to the interior of the liquid vessel 16 is used up without even a single drop being wasted. If the liquid is inexpensive, as in the case of water or a physiological saline solution, there is no particular inconvenience if some is left. If the liquid is a costly medicine, however, using up all of the liquid in the manner described above is economical since waste is avoided.

## Claims

1. An ultrasonic atomizer having:
a liquid vessel (16) for accommodating a liquid to be atomized; and
an ultrasonic pump (12) comprising a pump shaft (21) having a lower end situated inside said liquid vessel (16) and formed to have a pump bore (22) passing through the pump shaft (21) axially and having open upper and lower ends, and an ultrasonic vibrator (23) mounted on the pump shaft (21);
**characterized in that** a lower end of said pump shaft (21) is disposed in close proximity to a bottom surface or side surface of said liquid vessel (16) in such a manner that residual liquid remaining inside said liquid vessel (16) is pumped upon attaching itself to the lower end of the pump shaft (21) by surface tension.

2. An ultrasonic atomizer according to claim 1, wherein said liquid vessel (16) is formed to have a recess (66) for collecting the residual liquid remaining inside said liquid vessel (16), and the lower end of said pump shaft (21) is disposed so as to face said recess (66).

3. An ultrasonic atomizer according to claim 1 or 2, wherein said liquid vessel (16) is freely attachable and detachable.

## Patentansprüche

1. Ultraschallzerstäuber mit
einem Flüssigkeitsbehälter (16) zur Aufnahme einer zu zerstäubenden Flüssigkeit; und
einer Ultraschallpumpe (12), welche einen Pumpenschaft (21), der mit seinem unteren Ende im Flüssigkeitsbehälter (16) liegt und so ausgebildet ist, dass er eine durch den Pumpenschaft (21) axial verlaufende Pumpenbohrung (22) aufweist, und der obere und untere Enden aufweist, und einen an dem Pumpenschaft (21) angebrachten Ultraschallschwingungserzeuger (23) umfasst;
**dadurch gekennzeichnet, dass** ein unteres Ende des Pumpenschafts (21) in großer Nähe zu einer Bodenfläche oder Seitenfläche des Flüssigkeitsbehälters (16) in einer solchen Weise angeordnet ist, dass in dem Flüssigkeitsbehälter (16) verbleibende Restflüssigkeit, indem sie sich durch Oberflächenspannung an das untere Ende des Pumpenschafts (21) anhängt, gepumpt wird.

2. Ultraschallzerstäuber nach Anspruch 1, wobei der Flüssigkeitsbehälter (16) mit einer Ausnehmung (66) zum Sammeln der in dem Flüssigkeitsbehälter (16) verbleibenden Restflüssigkeit ausgebildet ist und das untere Ende des Pumpenschafts (21) der Ausnehmung (66) zugekehrt angeordnet ist.

3. Ultraschallzerstäuber nach Anspruch 1 oder 2, wobei der Flüssigkeitsbehälter (16) frei anbringbar und abnehmbar ist.

## Revendications

1. Atomiseur à ultrasons comprenant :
une cuve à liquide (16) pour loger un liquide destiné à être atomisé ; et
une pompe à ultrasons (12) comprenant un arbre de pompe (21) présentant une extrémité inférieure située à l'intérieur de ladite cuve à liquide (16) et formée pour présenter un orifice de pompe (22) traversant axialement l'arbre de pompe (21) et présentant des extrémités supérieure et inférieure ouvertes, et un vibrateur à ultrasons (23) monté sur l'arbre de pompe (21) ;
**caractérisé en ce qu'**une extrémité inférieure dudit arbre de pompe (21) est disposée directement à proximité d'une surface de fond ou d'une surface latérale de ladite cuve à liquide (16), de telle sorte que le liquide résiduel demeurant à l'intérieur de ladite cuve à liquide (16) soit pompé en se fixant à l'extrémité inférieure de l'arbre de pompe (21) par la tension superficielle.

2. Atomiseur à ultrasons selon la revendication 1, dans lequel ladite cuve à liquide (16) est formée pour présenter un évidement (66) pour collecter le liquide résiduel demeurant à l'intérieur de la cuve à liquide (16), et l'extrémité inférieure dudit arbre de pompe (21) est disposée de manière à faire face audit évidement (66).

3. Atomiseur à ultrasons selon la revendication 1 ou 2, dans lequel ladite cuve à liquide (16) peut être fixée ou détachée librement.
